# EUROPÄISCHE PATENTSCHRIFT

(11) **EP 4 112 108 B1**
(45) Veröffentlichungstag und Bekanntmachung des Hinweises auf die Patenterteilung: **27.11.2024**
(21) Anmeldenummer: 22192630.6
(22) Anmeldetag: 09.09.2020
(51) Int. Cl.: A61M 15/00, A61M 11/00, A61M 16/00

(54) **VERNEBLERSYSTEM**
NEBULIZER SYSTEM
SYSTÈME DE NÉBULISEUR

(43) Veröffentlichungstag der Anmeldung: 04.01.2023
(62) Teilanmeldung aus: 20195298.3
(73) Patentinhaber: Pulmotree Medical GmbH, 80333 München (DE)
(72) Erfinder: GÖRSTNER, Pia, 80335 München (DE); KELLNER, Kristian, 80804 München (DE); KRÜGER, Ulf, 80799 München (DE)
(74) Vertreter: Stolmár & Partner Patentanwälte PartG mbB

(56) Entgegenhaltungen:
- EP-A1- 1 772 166
- EP-A1- 3 581 038
- EP-A2- 3 431 132
- EP-B1- 2 062 608
- WO-A1-2019/186150
- WO-A1-2020/072478
- WO-A1-2020/104379
- WO-A2-2012/026963
- WO-A2-2014/165694
- US-A1- 2013 158 474
- US-A1- 2017 304 563
- US-A1- 2019 298 980
- US-A1- 2020 113 246
- US-A1- 2020 206 415

## Beschreibung

Die Erfindung betrifft ein Verneblersystem für einen Mesh-Vernebler gemäß Anspruch 1. Bevorzugte Ausführungsformen sind in den abhängigen Ansprüchen definiert.

Als Vernebler werden Medizingeräte bezeichnet, mit deren Hilfe flüssige Therapeutika in die Atemwege eines Patienten eingebracht werden. Dazu umfassen gängige Vernebler einen Verneblerkopf, in welchem das Therapeutikum vernebelt und einem vom Patienten eingeatmeten Luftstrom zugegeben wird. Es sind unterschiedliche Arten von Verneblerköpfen bekannt, welche in der Regel nach einem der folgenden Prinzipien funktionieren:
Düsenvernebler: Bei dieser Art von Verneblerköpfen wird ein unter Druck stehendes Gas mittels einer Düse durch ein Reservoir des Therapeutikums geleitet, wodurch das Therapeutikum zu einem Nebel zerstäubt wird. Entsprechende Verneblerköpfe sind jedoch unhandlich und geräuschintensiv, so dass sie nur noch selten eingesetzt werden.
Ultraschallvernebler: Bei dieser Art von Verneblerköpfen wird das Therapeutikum über einen Ultraschall-Vibrator geleitet, welcher das Therapeutikum zu einem Nebel zerstäubt.
Mesh-Vernebler: Bei dieser Art von Verneblerköpfen wird das Therapeutikum durch ein mit Ultraschall beaufschlagtes Gitternetz zerstäubt, welches die Flüssigkeitsoberfläche des Therapeutikums bedeckt.

Weiterhin umfassen Vernebler eine Dosiereinheit, mittels welcher eine vorgegebene oder vorgebbare Menge eines Therapeutikums dem Verneblerkopf zugeführt wird. Hier sind folgende Prinzipien bekannt:
Offene Dosierung: Die Dosiereinheit kann als offene Dosiereinheit ausgeführt sein, welcher das Therapeutikum manuell aus Portionsampullen oder Dosierpipetten zugeführt wird. Hierbei ist die Handhabung zumeist umständlich, zudem besteht die Gefahr von Fehldosierungen. Fehldosierungen können durch unvollständige Entleerung von Portionsampullen (Unterdosierung) oder durch Fehlbedienung einer Dosierpipette (Über- oder Unterdosierung) verursacht werden.
Geschlossene Dosierung: Bei geschlossenen Dosiereinheiten werden in der Regel Portionsampullen mit dem Therapeutikum in die Dosiereinheit eingelegt und dort durch einen Mechanismus aufgestochen und entleert. Auch bei bekannten geschlossenen Dosiereinheiten ist die Handhabung noch nicht optimal. Zudem entstehen bei der Verwendung von Portionsampullen (sowohl bei offenen als auch bei geschlossenen Dosiereinheiten) erhebliche Mengen an Verpackungsabfällen.

Sowohl der Verneblerkopf als auch die Dosiereinheit von Verneblern können nur über einen begrenzten Zeitraum verwendet werden, da ihre Funktionssicherheit durch Verschleiß und/oder Verschmutzung beeinträchtigt wird. Insbesondere Verschmutzungsablagerungen, z.B. aus dem zugeführten Atemluftstrom, können in Verbindung von Feuchtigkeit einen Nährboden für Krankheitserreger bilden. Derzeit verfügbare Verneblerköpfe und Dosiereinheiten lassen sich nicht unkompliziert bzw. nicht ausreichend gründlich reinigen. Zudem lässt die generelle Performance von Dosiereinheiten und Verneblerköpfen verschleißbedingt mit der Zeit nach. Daher müssen die jeweiligen Komponenten regelmäßig ausgetauscht werden. Es gibt daher Verneblersysteme, welche eine Basiseinheit sowie mehrere Verneblerköpfe und/oder Dosiereinheiten umfassen, welche zusammen mit der Basiseinheit verwendet werden können.

Vernebler umfassen weiterhin eine Steuerungseinheit, welche den Verneblerkopf und/oder die Dosiereinheit kontrolliert. Die Steuerungseinheit ist in der Regel in der Basiseinheit angeordnet und verfügt über einen oder mehrere Sensoren, welche z.B. die Geschwindigkeit eines Luftstroms durch den Verneblerkopf bestimmen. Die Steuerungseiheit kann dann bei Vorliegen eines ausreichenden Luftstroms den Verneblerkopf aktivieren, um das Therapeutikum zu zerstäuben.

Einige Vernebler verfügen über eine Kommunikationsschnittstelle, um Messwerte der Sensoren an ein externes Steuerungs- oder Visualisierungsgerät zu übertragen. Diese Messwerte können dann genutzt werden, um dem Patienten eine Rückmeldung über sein Nutzungsverhalten und mögliche Verbesserungen zu geben.

Generell besteht ein Bedürfnis, Vernebler bereitzustellen, deren Handhabung, Funktion, und/oder Funktionssicherheit verbessert ist.

Gemäß eines ersten Aspekts wird diese Aufgabe gelöst durch einen Vernebler mit einer Dosiereinheit zur Bereitstellung einer vorgegebenen Menge eines Therapeutikums, wobei das Therapeutikum in einem mittels Druck zu öffnenden Portionsbehälter zugeführt wird; wobei die Dosiereinheit eine Aufnahmekammer mit einer Dosieröffnung zur Abgabe des Therapeutikums, einer Öffnung zum Einführen eines Portionsbehälters und einen Verschlusskörper für die Öffnung aufweist; wobei der Verschlusskörper eingerichtet ist, von einer offenen Position, in welcher der Verschlusskörper die Öffnung freigibt, in einer geschlossene Position bewegt zu werden, in welcher der Verschlusskörper die Öffnung verschließt; und wobei der Verschlusskörper ferner eingerichtet ist, von der geschlossenen Position in eine Entleerungsposition bewegt zu werden, in welcher der Verschlusskörper einen Druck auf den Portionsbehälter ausübt, um diesen zu öffnen und das Therapeutikum freizusetzen.

Der Portionsbehälter kann beispielsweise eine Oberschale, eine Unterschale, sowie eine die Oberschale mit der Unterschale verbindende Verschlussnaht aufweisen. Zur Freisetzung des Therapeutikums kann der Portionsbehälter durch den Druck entlang der Verschlussnaht aufreißen, so dass das Therapeutikum austreten kann. Der Portionsbehälter kann auch nur entlang eines Teils der Verschlussnaht, oder an anderen Stellen der Ober- und oder Unterschale aufreißen. Ggf. können hierzu Schwächungslinien in die jeweiligen Materialabschnitte eingebracht sein.

Die Bewegung des Verschlusskörpers von der offenen Position in die geschlossene Position kann eine lineare Bewegung sein oder umfassen. Beispielsweise kann der Verschlusskörper eine Verschlusskappe sein, die von oben auf die Dosiereinheit aufgesetzt wird.

Die Bewegung des Verschlusskörpers von der offenen Position in die geschlossene Position kann eine rotierende Bewegung sein oder umfassen. Dazu kann der Verschlusskörper von oben auf die Dosiereinheit aufgeschraubt werden.

Die Bewegung des Verschlusskörpers von der geschlossenen Position in die Entleerungsposition kann ebenfalls eine rotierende Bewegung sein oder umfassen.

Dazu können die Dosiereinheit und der Verschlusskörper bajonettartige Führungselemente aufweisen, welche eine lineare Bewegung des Verschlusskörpers von der offenen in die geschlossene Position ermöglichen, und weiter eine rotierende Bewegung des Verschlusskörpers von der geschlossenen Position in die Entleerungsposition ermöglichen. Die Führungselemente können einen oder mehrere Führungsstifte aufweisen, welche mit einer oder mehreren Führungsnuten des jeweils anderen Elements zusammenwirken. Die Führungsnuten können einen linearen Abschnitt und einen geneigten umlaufenden Abschnitt aufweisen. Über den Neigungswinkel des umlaufenden Abschnitts lässt sich konstruktiv die zur Öffnung des Portionsbehälters erforderliche Kraft bestimmen.

Alternativ können die Dosiereinheit und der Verschlusskörper zusammenwirkende Gewindeabschnitte aufweisen, wobei der Verschlusskörper durch eine Schraubbewegung zunächst von der offenen in die geschlossene Position und dann weiter in die Entleerungsposition bewegt werden kann. Auch hier die zum Öffnen des Portionsbehälters erforderliche Kraft konstruktiv über die Steigung der Gewindeabschnitte bestimmt werden.

Die Bewegung des Verschlusskörpers von der offenen in die geschlossene Position und/oder von der geschlossenen Position in die Entleerungsposition kann eine Schwenkbewegung umfassen. Dazu kann der Verschlusskörper über ein Gelenk mit der Dosiereinheit verbunden sein.

In einer möglichen Ausführungsform eines Verneblers kann die Dosiereinheit eine Entnahmehilfe für entleerte Portionsbehälter aufweisen.

Dabei kann Entnahmehilfe Halteelemente umfassen, welche bei der Bewegung des Verschlusskörpers von der offenen in die geschlossene Position und/oder von der geschlossenen Position in die Entleerungsposition den Portionsbehälter greifen, und diesen bei der Bewegung des Verschlusskörpers zurück in die offene Position festhalten. Der entleerte Portionsbehälter kann somit automatisch mit dem Verschlusskörper entnommen werden.

Die Entnahmehilfe kann weiterhin Auswurfmittel umfassen, welche den Portionsbehälter aus den Halteelementen lösen. Damit kann der entleerte Portionsbehälter anschließend einfach freigegeben werden.

In einer weiteren möglichen Ausführung kann die Entnahmehilfe eine seitliche Ausnehmung der Aufnahmekammer umfassen, durch welche eine Handhabungszunge des Portionsbehälters geführt bzw. führbar ist. Die Handhabungszunge kann dann nach Anwendung des Verneblers einfach gegriffen werden, um den Portionsbehälter aus der Aufnahmekammer zu entnehmen.

In noch einer weiteren Ausführung kann die Entnahmehilfe einen Auswurfhebel umfassen, welcher den Portionsbehälter auf einer der Öffnung der Aufnahmekammer entgegengesetzten Seite hintergreift. Dadurch kann der Portionsbehälter in der Aufnahmekammer angehoben werden, um ihn anschließend leicht entnehmen zu können.

Gemäß eines weiteren Aspekts wird die Aufgabe gelöst durch einen Vernebler mit einem Verneblerkopf und einer Steuerungseinheit, wobei die Steuerungseinheit wenigstens einen Sensor umfasst und eingerichtet ist, den Verneblerkopf in Abhängigkeit von Messwerten des wenigstens einen Sensors zu aktivieren, wobei der Vernebler einen Signalgeber umfasst der eingerichtet ist, ein für den Patienten unmittelbar wahrnehmbares Signal auszugeben, welches einen Aktivierungszustand des Verneblerkopfes, ein Messwert des wenigstens einen Sensors, und/oder hiervon abgeleitete Informationen anzeigt.

Durch den Signalgeber kann der Patient ein unmittelbares Feedback über den Aktivierungszustand des Verneblerkopfes oder weitere, ggf. therapierelevante Informationen erhalten.

Der Signalgeber kann einen akustischen Signalgeber umfassen, beispielsweise einen Lautsprecher zur Abgebe von Signaltönen. Ebenso kann der Signalgeber einen optischen Signalgeber umfassen, z.B. eine oder mehrere Signalleuchten oder LEDs.

In einer möglichen Ausführung eines Vernebler kann der Signalgeber einen haptischen Signalgeber umfassen, dies kann ein Vibrationserreger sein.

Der Vibrationserreger kann an mit einer Innenseite eines Gehäuses des Verneblers verbunden sein. Vernebler sind oft so ausgeführt, dass sei ein mit einer Hand zu umfassendes Gehäuse aufweisen. Durch Verbindung des Vibrationserregers mit dem Gehäuse kann die Vibration durch den Patienten besonders unmittelbar wahrgenommen werden. Der Vibrationserreger kann dabei direktam Gehäuse angebracht sein, oder auch entfernt von dem Gehäuse angeordnet und über ein Koppelelement mit diesem verbunden sein.

Dabei kann das Gehäuse des Verneblers so ausgeführt sein, dass eine Fortpflanzung der Vibration entlang des Gehäuses reduziert oder ganz verhindert wird. Dazu kann das Gehäuse in der Umgebung der Stelle, mit welcher der Vibrationserreger verbunden ist, eine erhöhte Elastizität aufweisen. Hierdurch wird erreicht, dass sich die Vibration nicht entlang des Gehäuses zu Stellen überträgt, wo sie unerwünscht ist. Dies trifft beispielsweise auf ein Mundstück des Verneblers zu, welches von dem Patienten mit den Lippen oder mit den Zähnen berührt werden kann. Hier würde eine Vibration ggf. als unangenehm empfunden werden.

In einer möglichen Ausführung kann der Signalgeber eingerichtet sein, wenigstens zwei unterschiedliche Signale auszugeben. Hierdurch können dem Patienten mehr Informationen zur Verfügung gestellt werden.

Beispielsweise kann der Vibrationserreger eingerichtet sein, Vibrationssignale mit unterschiedlichen Frequenzen und/oder Intensitäten abzugeben.

Gemäß eines weiteren Aspekts wird die Aufgabe gelöst durch ein Verneblersystem für einen Mesh-Vernebler mit einer Basiseinheit und wenigstens einem Verneblerkopf und/oder wenigstens einer Dosiereinheit, welche mit der Basiseinheit kombiniert werden können, um einen funktionsbereiten Vernebler zu bilden, wobei die wenigstens eine Dosiereinheit, ein in die Dosiereinheit einzulegender Portionsbehälter, und/oder der wenigstens eine Verneblerkopf mit einem Identifikationsmerkmal ausgerüstet sind, wobei die Basiseinheit eine Steuerungseinheit umfasst, welche eingerichtet ist, das Identifikationsmerkmal eines Verneblerkopfes, einer Dosiereinheit, und/oder eines Portionsbehälters zu registrieren, und wobei ferner die Steuerungseinrichtung eingerichtet ist, anhand des Identifikationsmerkmals festzustellen, ob der Betrieb des Verneblerkopfes, der Dosiereinheit, und/oder des Portionsbehälters mit der Basiseinheit zulässig ist.

Die Steuerungseinheit kann durch die entsprechende Ausführung feststellen, ob das Verneblersystem mit unzulässigen Komponenten betrieben werden soll.

Wenn die Steuerungseinheit weiterhin eingerichtet ist, den Verneblerkopf und/oder die Dosiereinheit nur dann zu aktivieren, wenn diese mit der Basiseinheit verwendet werden dürfen, kann sichergestellt werden, dass die Betriebssicherheit des Verneblersystems nicht durch systemfremde Komponenten kompromittiert wird. Systemfremde Komponenten können hierbei Produktfälschungen oder Fremdherstellerprodukte sein, deren Verwendung in dem Verneblersystem nicht zugelassen oder nicht erwünscht ist.

In der Steuerungseinheit kann eine Liste mit zulässigen Identifikationsmerkmalen enthalten. Diese Liste kann ggf. über eine Datenverbindung verändert werden, um neue Komponenten für die Verwendung zuzulassen, oder die Verwendung von bislang zugelassenen Komponenten zukünftig zu verhindern.

Das Identifikationsmerkmal kann einen RFID-Chip umfassen, und die Steuerungseinheit einen RFID-Leser umfassen. Durch diese Ausführung kann die Registrierung des Identifikationsmerkmals einfach und kontaktlos erfolgen.

Das Identifikationsmerkmal kann einen Speicherchip umfassen, und die Basiseinheit und der Verneblerkopf, die Dosiereinheit, und/oder der Portionsbehälter können so ausgeführt sein, dass bei der Kombination des Basiseinheit mit dem Verneblerkopf, der Dosiereinheit, und/oder dem Portionsbehälter eine galvanische Verbindung zwischen der Steuerung und dem Speicherchip hergestellt wird. Hierzu können die Basiseinheit und der Verneblerkopf bzw. die Dosiereinheit Steck- oder Federkontakte aufweisen, über welche die Verbindung hergestellt wird.

In einer möglichen Ausführung kann das Identifikationsmerkmal Informationen über eine Haltbarkeit und/oder eine maximale Verwendungsdauer des Verneblerkopfes, der Dosiereinheit, und/oder des Portionsbehälters beinhalten. Die Steuereinheit kann dann zusätzlich zu der grundsätzlichen Eignung des Verneblerkopfes bzw. der Dosiereinheit und/oder des Portionsbehälters auch überprüfen, ob diese ggf. eine Haltbarkeits- und/oder Verwendungsdauer überschritten haben und somit nicht mehr sicher eingesetzt werden können.

Die Steuereinheit kann dabei eingerichtet sein, Nutzungsdaten auf den RFID-Chip oder den Speicherchip zurückzuschreiben. Auf diese Weise kann auch die Einhaltung einer vorgegebenen maximalen Anzahl von Verwendungen und/oder eine maximale Verwendungsdauer nach der ersten Verwendung durch die Steuereinheit überwascht werden.

Die Aufgabe wird auch gelöst durch einen Verneblerkopf für ein Verneblersystem, wobei der Verneblerkopf mit einem Identifikationsmerkmal ausgerüstet ist.

Weiter wird die Aufgabe gelöst durch eine Dosiereinheit und/oder durch einen Portionsbehälter für ein Verneblersystem, wobei die Dosiereinheit bzw. der Portionsbehälter mit einem Identifikationsmerkmal ausgerüstet sind.

Ebenso wird die Aufgabe gelöst durch eine Basiseinheit für ein entsprechendes Verneblersystem.

Die Offenbarung wird nachfolgend anhand einiger beispielhafter Figuren näher beschrieben, wobei die in dargestellten Ausführungsbeispiele lediglich zum besseren Verständnis der Offenbarung beitragen sollen, ohne diese einzuschränken.

Es zeigen:
Fig. 1: einen Vernebler
Fig. 2: einen Vernebler in einer Explosionsdarstellung,
Fig. 3a-c: Portionsbehälter
Fig. 4a-d: eine Dosiereinheit,
Fig. 5a, b: eine weitere Dosiereinheit,
Fig. 6: noch eine weitere Dosiereinheit,
Fig. 7: einen Verneblerkopf,
Fig. 8: eine Basis eines Verneblers,
Fig. 9a, b: Aktivierungsverläufe eines Vibrationserregers.

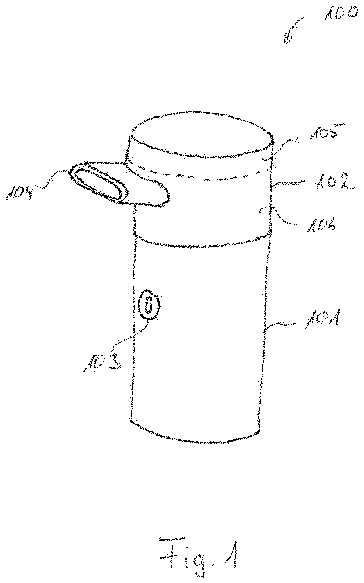

Figur 1 zeigt einen Vernebler 100. Der Vernebler 100 umfasst eine Basis 101 und einen Kopf 102. Die Basis 101 und der Kopf 102 des Verneblers weisen eine im Wesentlichen kreiszylindrische Kontur, welche leicht mit einer Hand umfasst werden kann.

An der Basis 101 des Verneblers 100 ist ein Hauptschalter 103 angeordnet, mit welchem der Vernebler 100 ein- und ausgeschaltet werden kann. Am Kopf 102 des Verneblers 100 ist ein Mundstück 104 angeordnet.

Der Kopf 102 des Verneblers 100 umfasst eine Dosiereinheit 105 und den eigentlichen Verneblerkopf 106, welcher einen Aerosolgenerator beinhaltet. Dosiereinheit 105 und Verneblerkopf 106 sind im dargestellten Beispiel als einheitliche Baugruppe dargestellt, sie können jedoch auch als getrennte Baugruppen ausgeführt sein.

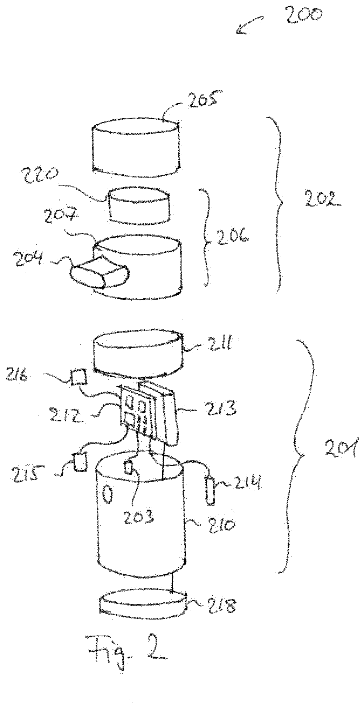

In Figur 2 ist ein Vernebler 200 in einer Explosionsdarstellung abgebildet. Der Vernebler 200 umfasst wiederum eine Basis 201 und einen Kopf 202.

Die Basis 201 des Verneblers 200 umfasst ein unteres Gehäuseteil 210 sowie ein oberes Gehäuseteil 211. In dem unteren Gehäuseteil 210 sind eine Steuereinheit 212 sowie ein Energiespeicher 213 aufgenommen. Der Energiespeicher 213 kann einen Akkumulator, beispielsweise einen Li-lonen-Akkumulator sein.

Die Steuereinheit 212 ist mit dem Hauptschalter 203 verbunden, und kann darüber ein- und ausgeschaltet werden. Weiterhin ist die Steuereinheit 212 mit einer Antennenbaugruppe 214, einem Vibrationserreger 215, und einem Strömungssensor 216 verbunden. Die Antennenbaugruppe 214 dient dazu, eine Kommunikationsverbindung herzustellen, beispielsweise ein Bluetooth-, Mobilfunk- oder WiFi-Verbindung. Die Funktion der weiteren Komponenten wird später erläutert.

Die Basis 201 kann weiterhin eine Ladebaugruppe 218 umfassen, über welche der Energiespeicher 213 kontaktlos aufgeladen werden kann. Die Ladebaugruppe 218 kann das untere Gehäuseteil 210 nach unten abschließen.

Das obere Gehäuseteil 211 schließt die Basis 201 dichtend ab, so dass die elektronischen Komponenten darin vor Feuchtigkeit geschützt sind. Gleichzeitig stellt das obere Gehäuseteil 211 eine Verbindung zum Kopf 202 des Verneblers 200 her.

Der Kopf 202 des Verneblers 200 umfasst eine Dosierbaugruppe 205 und einen Verneblerkopf 206. Der Verneblerkopf 206 besteht aus einem Kopfgehäuse 207, an welchem ein Mundstück 204 angeordnet ist, und einen Aerosolgenerator 220, der in dem Kopfgehäuse 207 angeordnet ist. Obwohl das Mundstück 204 als einteilig mit dem Kopfgehäuse 207 dargestellt ist, kann das Mundstück 204 auch als separates Bauteil ausgeführt sein, welche über geeignete Verbindungsmittel mit dem Kopfgehäuse 207 verbunden ist. Dazu kann beispielsweise das Kopfgehäuse 207 einen Stutzen aufweisen, auf welchen das Mundstück 204 aufgesteckt wird.

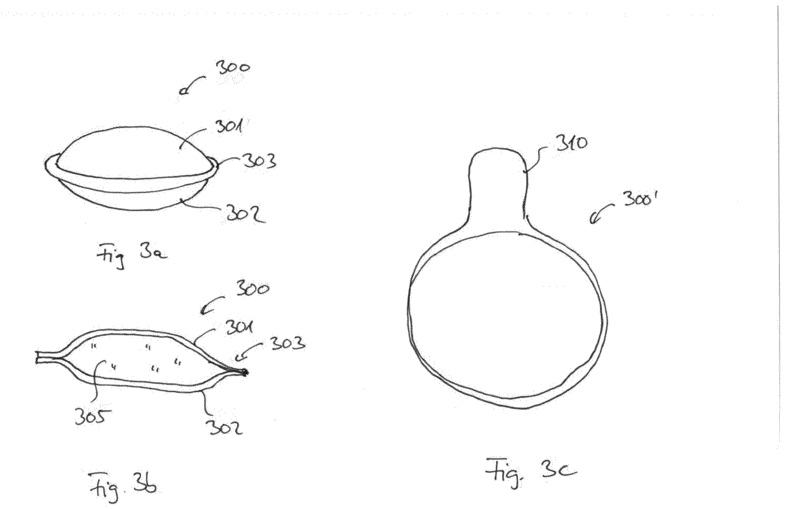

Die Dosiereinheit 205 dient dazu, eine definierte Menge eines Therapeutikums an den Aerosolgenerator 220 abzugeben. Im Gegensatz zu bekannten Dosiereinheiten, die eine manuelle Zudosierung der gewünschten Menge des Therapeutikums erfordern, ist die Dosiereinheit 205 dazu eingerichtet, vorbefüllte Portionsbehälter mit dem Therapeutikum aufzunehmen und zu verarbeiten. Mögliche Ausführungen solcher Portionsbehälter sind in den Figuren 3a bis 3c dargestellt.

Figur 3a zeigt einen Portionsbehälter 300 in einer perspektivischen Darstellung, Figur 3b zeigt den gleichen Portionsbehälter 300 in einer Schnittdarstellung. Der Portionsbehälter 300 umfasst eine Oberschale 301 und eine Unterschale 302, welche entlang einer Verschlussnaht 303 verbunden sind. Zwischen der Oberschale 301 und der Unterschale 302 ist ein Volumen 305 gebildet, welches mit dem Therapeutikum gefüllt ist.

Die Oberschale 301 und die Unterschale 302 können beispielsweise aus einer Metallfolie bestehen. Alternativ können die Ober- und Unterschale 301, 302 auch aus einer Kunststofffolie, beispielsweise aus einem thermoplastischen Polymer, bestehen. Beispiele solcher Polymere sind Polyethylen (PE), Polyethylenterephthalat (PET), oder Polyetheretherketon (PEEK).

Die Verschlussnaht 303 kann beispielsweise durch Verkleben, Verpressen, oder Verschweißen der Oberschale 301 und der Unterschale 302 hergestellt werden.

Figur 3c zeigt eine alternative Ausführung eines Portionsbehälters 300`. Anders als der Portionsbehälter 300 weist der Portionsbehälter 300 eine Handhabungszunge 310 auf, deren Funktion später erläutert wird.

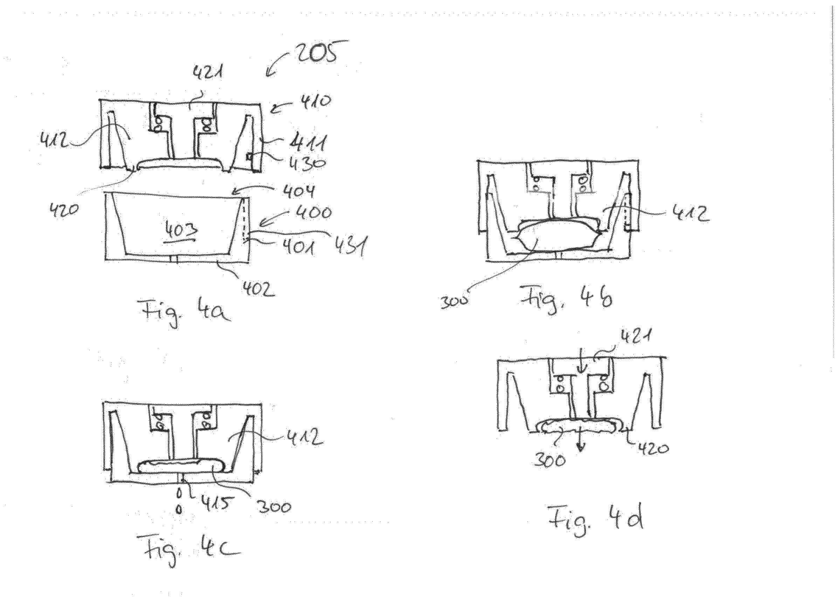

Die Figuren 4a-d zeigen einen möglichen Aufbau der Dosiereinheit 205. Die Dosiereinheit 205 umfasst einen Aufnahmekörper 400 mit einer umlaufenden Wandung 401 sowie einem Boden 402. Wandung 401 und Boden 402 bilden eine Aufnahmekammer 403 mit einer nach oben weisenden Öffnung 404, durch welche ein nicht dargestellter Portionsbehälter in die Aufnahmekammer 403 eingeführt werden kann.

Die Dosiereinheit 205 umfasst weiterhin einen Verschlusskörper 410 in Form eines Deckels, welcher auf den Aufnahmekörper 400 aufgesetzt werden kann, um die Öffnung 404 zu verschließen. Dazu weist der Verschlusskörper 410 einen Kragen 411 auf, welcher die Wandung 401 umgreift.

Der Verschlusskörper 410 umfasst weiterhin einen Verdrängerkörper 412, der so ausgeführt ist, dass ein bei Aufsetzen des Verschlusskörpers 410 auf den Aufnahmekörper 400 in die Aufnahmekammer 403 eintaucht.

In Figur 4a ist die Dosiereinheit 205 in einer geöffneten Position dargestellt. Figur 4b zeigt die Dosiereinheit in einer geschlossenen Position mit eingelegtem Portionsbehälter 300. In der geschlossenen Position liegt der Verdrängerkörper 412 an dem Portionsbehälter an.

Figur 4c zeigt die Dosiereinheit 305 in einer Entleerungsposition. In dieser Position taucht der Verdrängerkörper 412 tiefer in die Aufnahmekammer 403 ein und drückt dabei auf den Portionsbehälter 300, wodurch dieser aufreißt und sich entleert. Die Stelle, an welcher der Portionsbehälter 300 aufreißt, kann konstruktiv vorgegeben sein. Dazu könen die Ober- und/oder Unterschale 301, 302 nicht dargestellte Schwächungslinien aufweisen. Das Therapeutikum kann dann durch eine Dosieröffnung 415 ablaufen und in den nicht dargestellten Verneblerkopf gelangen.

Um den entleerten Portionsbehälter aus der Aufnahmekammer 403 zu entnehmen, weist der Verschlusskörper 410 eine Entnahmehilfe in Form von Greifhaken 420 auf, welche den Portionsbehälter 300 umgreifen, wenn der Verschlusskörper 410 in die Entleerungsposition bewegt wird.

Wird der Verschlusskörper 410 anschließend wieder in die geöffnete Position bewegt, nimmt dieser den entleerten Portionsbehälter 300 mit, dies ist in Figur 4d dargestellt.

Um den entleerten Portionsbehälter 300 aus den Greifhaken 420 zu lösen, weist der Verschlusskörper 410 einen federgelagerten Auswurfstößel 421 auf. Dieser kann durch einen Druck auf die Oberseite des Auswurfstößels bewegt werden, um den Portionsbehälter 300 aus den Greifhaken 420 zu drücken.

Um den Verschlusskörper 410 von der geöffneten Position in die geschlossene Position und in die Entleerungsposition zu bewegen, kann eine bajonettartige Führung vorgesehen sein. Dazu ist in dem Kragen 411 ein Pin 430 angeordnet, der in einer Führungsnut 431 in der Wandung 401 läuft. Die Führungsnut 430 verläuft vom oberen Rand der Wandung 401 gerade nach unten bis zu einer Tiefe, die der geschlossenen Position entspricht. Weiter verläuft die Führungsnut 431 dann in einem Winkel, so dass der Verschlusskörper 410 in einer schraubenden Bewegung in die Entleerungsposition bewegt werden kann. Über die Steigung der Führungsnut 431 in diesem Abschnitt lässt sich die zum Entleeren des Portionsbehälters 300 erforderliche Kraft bestimmen.

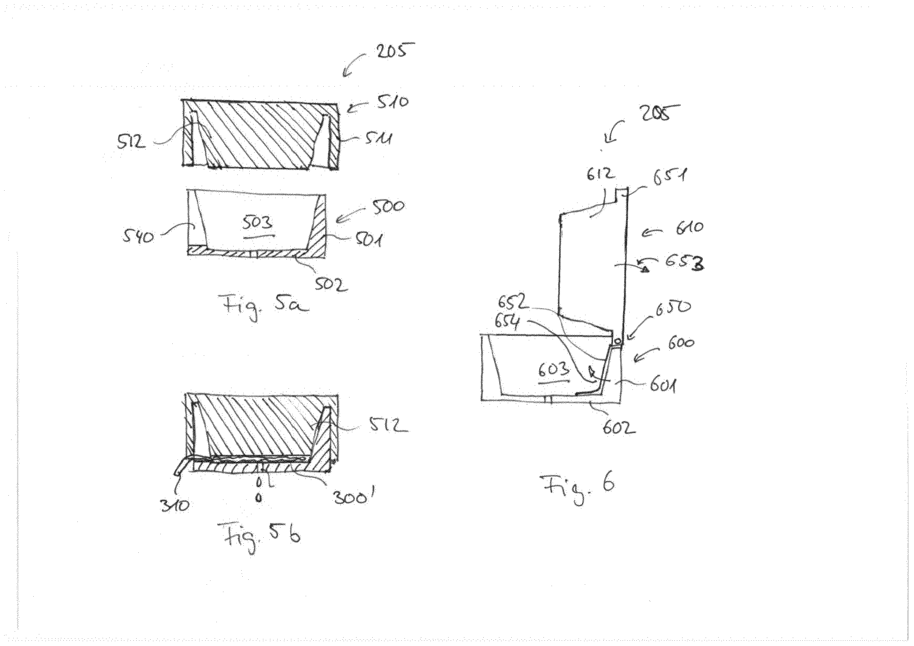

In den Figuren 5a, 5b ist eine alternative Ausführung der Dosiereinheit 205 dargestellt, wobei gleichartige Komponenten mit einem um 100 erhöhten Bezugszeichen versehen sind. Die Wandung 501 des Aufnahmekörpers 500 weist hier eine seitliche Ausnehmung 540 auf, durch welche eine Handhabungszunge 310 des Portionsbehälters 300' geführt werden kann. Diese Handhabungszunge 310 kann genutzt werden, um den entleerten Portionsbehälter 300` wieder aus der Aufnahmekammer 503 zu entnehmen.

In Figur 6 ist eine weitere mögliche Ausführungsform der Dosiereinheit 205 dargestellt, in welcher der Verschlusskörper 610 in einer Schwenkbewegung zwischen der geöffneten, der geschlossenen, und der Entleerungsposition bewegt werden kann. Dazu ist der Verschlusskörper mit einem Gelenk 650 an dem Aufnahmekörper befestigt.

In der Ausführung der Figur 6 weist der Verschlusskörper 610 keinen Kragen auf, sondern lediglich den Verdrängerkörper 612 und einen Anschlagrand 651.

Als Entnahmehilfe für entleerte Portionsbehälter weist die Dosiereinheit 205 in dieser Ausführung einen Auswurfhebel 652 auf, welcher den nicht dargestellten Portionsbehälter anhebt. Dazu kann der Auswurfhebel 652 so mit dem Verschlusskörper 612 gekoppelt sein, dass er sich durch eine Schwenkbewegung des Verschlusskörpers 610 über die geöffnete Position hinaus automatisch anhebt (Pfeile 653, 654).

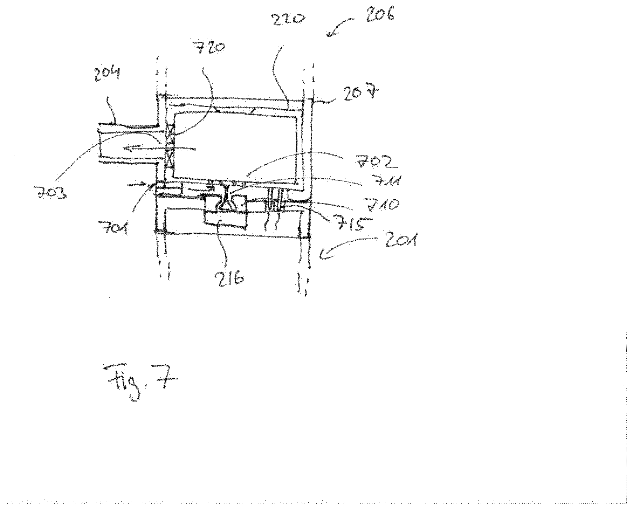

In Figur 7 ist ein Verneblerkopf 206 schematisch dargestellt. Der Verneblerkopf 206 umfasst ein Kopfgehäuse 207 mit Mundstück 204, sowie einen Aerosolgenerator 220. Der Innere Aufbau des Aerosolgenerators 220 ist aus Gründen der Übersichtlichkeit nicht dargestellt, es kann sich hier beispielsweise um einen Ultraschallvernebler oder einen Mesh-Vernebler handeln.

Der Luftstrom in dem Verneblerkopf 206 verläuft wie folgt: Luft strömt durch Zuluftöffnungen 701 im Bereich des Mundstücks 204 in den Verneblerkopf 206 ein, und tritt dann durch Eintrittsöffnungen 702 von unten mittig in den Aerosolgenerator 220 ein. Nach Vermischung mit dem Aerosol tritt die Luft durch eine Austrittsöffnung 703 des Aerosolgenerators 220 in das Mundstück 204, und wird dann vom Patienten eingeatmet.

Die Zuluftöffnungen 701 sind im Bereich des Mundstücks 204 angeordnet, um ein versehentliches Blockieren der Zuluftöffnungen 701 bei der Verwendung des Verneblers zu verhindern.

Da der Luftstrom durch einen vom Patienten erzeugten Unterdruck angetrieben wird, entsteht unter dem Aerosolgenerator 220 ebenfalls ein Unterdruck, welcher etwa proportional von dem Volumenstrom der eingeatmeten Luft ist. Dieser Unterdruck wird durch den Strömungssensor 216 gemessen, welcher unterhalb des Verneblerkopfes 206 in der Basis 201 angeordnet ist.

Zwischen dem Verneblerkopf 206 und dem Sensor 216 ist ein Ventil 710 angeordnet. Das Ventil 710 wird durch einen Pin 711 geöffnet, der mittig unter dem Aerosolgenerator 220 angebracht ist. Daas Ventil 710 verhindert eine Verschmutzung des Sensors 216, wenn der Verneblerkopf 206 nicht auf die Basis 201 aufgesetzt ist.

Ein weiteres, nicht dargestelltes Ventil kann vorgesehen sein, um einen entgegen der vorgesehenen Richtung durch den Verneblerkopf 206 verlaufenden Luftstrom zu unterbinden, beispielsweise falls ein Benutzer versehentlich in das Mundstück 204 bläst. Das zusätzliche Ventil ist vorzugsweise ein Einwegventil, also ein Ventil, welches einen Luftstrom nur in eine Richtung passieren lässt, beispielsweise ein Lippenventil.

Steckkontakte 715 des Aerosolgenerators 220 verbinden diesen mit der in Figur 7 nicht dargestellten Steuereinrichtung, sie dienen zur Aktivierung und Versorgung des Aerosolgenerators 220.

Der Aerosolgenerator 720 trägt einen RFID-Chip 720, welcher im dargestellten Ausführungsbeispiel ringförmig um die Austrittsöffnung 703 angeordnet, wobei die Form des RFID-Chips 720 hauptsächlich durch dessen Antenne bestimmt ist. Der RFID-Chip 720 kann auch an anderen Stellen des Aerosolgenerators 220 angeordnet sein. Zum Schutz vor Feuchtigkeit kann der RFID-Chip mit Silikon ummantelt sein.

Anstelle des RFID-Chips 720 kann der Aerosolgenerator 220 auch einen galvanisch gekoppelten Speicherchip umfassen. Dieser kann beispielsweise über die Steckkontakte 715 mit der Steuereinrichtung verbunden sein.

Der RFID-Chip 720 bzw. der Speicherchip enthält Identifikationsmerkmale des Aerosolgenerators 220 bzw. des Verneblerkopfes 206. Diese können von der Steuereinrichtung ausgelesen werden, um festzustellen, ob der Verneblerkopf 206 mit der Basis 201 kompatibel ist und verwendet werden darf. Weiterhin kann der RFID-Chip oder Speicherchip Daten über eine Haltbarkeit und/oder maximale Verwendungsdauer, sowie über die Anzahl bereits durchgeführter Verwendungen enthalten. Diese Daten können auch von der Steuereinheit nach einer Verwendung auf dem Chip gespeichert werden.

Die Steuereinrichtung nutzt auch diese Daten, um die Zulässigkeit der Nutzung, bzw. der weiteren Nutzung, des Verneblerkopfes mit der Basis 201 zu prüfen. Im Falle eines negativen Ergebnisses kann die Steuereinrichtung die Aktivierung des Verneblerkopfes unterlassen und ggf. eine Störungsmeldung ausgeben.

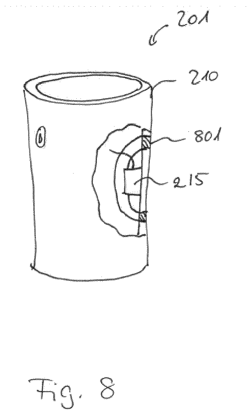

In Figur 8 ist ein Abschnitt des unteren Gehäuseteils 210 der Basis 201 eines Verneblers dargestellt, wobei ein Teil der Gehäusewand ausgebrochen dargestellt ist.

An einer Stelle des Gehäuseteils 210, die etwa gegenüber dem nicht dargestellten Mundstück liegt, ist der Vibrationserreger 215 an der Gehäusewand befestigt. Die Stelle ist so gewählt, dass eine durch den Vibrationserreger 215 erzeugte Vibration durch einen Benutzer des Verneblers gut wahrgenommen wird.

Um eine Ausbreitung der Vibration in Bereiche des Verneblers zu vermeiden, in denen eine Vibration unerwünscht ist, ist die Befestigungsstelle des Vibrationserregers 215 durch eine dämpfende Materialschicht 801 umgeben, welche die Vibrationen nicht oder nur gedämpft überträgt.

Zu Bereichen, in denen eine Vibration unerwünscht ist, gehört insbesondere das Mundstück.

Die dämpfende Materialschicht weist vorzugsweise eine höhere Elastizität auf als das umgebende Material, sie kann beispielsweise aus Silikon oder EPDM-Kautschuk bestehen.

Der Vibrationserreger 215 dient dazu, einem Benutzer des Verneblers ein Feedback über seine Nutzung zu geben.

Die Steuereinheit 212 aktiviert den Verneblerkopf 206 in Abhängigkeit von dem über den Sensor 216 gemessenen Atemstrom. Da der Benutzer diese Aktivierung jedoch nicht unmittelbar wahrnehmen kann, ist die korrekte Anwendung für den Benutzer schwer zu kontrollieren.

Um dies zu verbessern, kann die Steuereinrichtung 212 den Vibrationserreger gleichzeitig mit dem Verneblerkopf 2016 aktivieren, so dass der Benutzer eine direkte Rückmeldung erhält.

Neben der direkten Rückmeldung über die Aktivierung kann die Steuereinrichtung 212 dem Benutzer mittels des Vibrationserregers 215 auch weitere Informationen vermitteln.

Wenn beispielsweise eine Atemzugdauer des Benutzers nicht einer gewünschten Aktivierungsdauer des Verneblerkopfes 206 entspricht, so kann die Steuereinrichtung 212 den Vibrationserreger 215 nach Beendigung des Atemzugs weiter aktiviert lassen, um so dem Benutzer zu vermitteln, dass er länger einatmen sollte. Dabei kann die Dauer der weiteren Aktivierung durch die Steuerung 212 dynamisch angepasst werden, um den Benutzer sukzessive an die gewünschte Atemzugsdauer heranzuführen, ohne eine Frustration auszulösen. Ebenso kann eine dynamische Anpassung erfolgen, um den Benutzer langsam zu seiner physiologisch bedingten maximalen Atemzuglänge heranzuführen.

Die Steuerung 212 kann weiterhin dem Benutzer eine Rückmeldung geben, wenn der Atemzug zu schwach oder zu stark ist. Hierzu kann der Vibrationserreger 215 mit einer anderen Frequenz und/oder Intensität aktiviert werden.

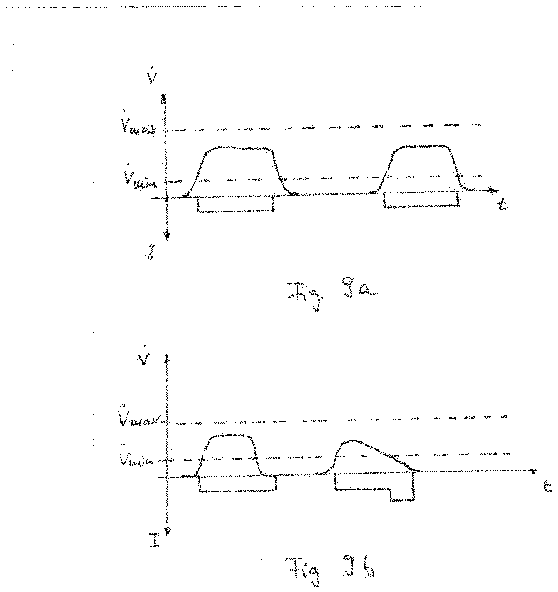

Mögliche Aktivierungsmuster des Vibrationserregers sind in den Figuren 9a, b dargestellt.

Figur 9a zeigt ein Diagramm mit zwei Atemzügen. Dabei ist auf der Längsachse die Zeit und auf der oberen Hochachse der Volumenstrom V` der eingeatmeten Luft aufgetragen, jeweils in willkürlichen Einheiten. Ein maximaler Volumenstrom V'ₘₐₓ und ein minimaler Volumenstrom V'ₘᵢₙ, die für die korrekte Funktion des Aerosolgenerators 220 erforderlich bzw. zulässig sind, sind ebenfalls eingetragen.

Auf der unteren Hochachse (unter der Längsachse ist der Aktivierungszustand I des Vibrationserregers 215 angezeigt. Bei beiden Atemzügen wird der Vibrationserreger 215 aktiviert, wenn der Volumenstrom V'ₘᵢₙ übersteigt, und nach Erreichen einer vorgegebenen Aktivierungszeit des Aerosolgenerators 220 deaktiviert.

In der Figur 9b ist ein ähnliches Diagramm mit zwei Atemzügen dargestellt. Dabei ist der erste Atemzug kürzer als die vorgesehen Aktivierungsdauer des Aerosolgenerators 220. Daher wird der Vibrationserreger 215 auch weiter aktiviert, nachdem der Atemzug bereits beendet ist.

Der zweite Atemzug im Diagramm der Figur 9b zeigt am Ende einen stark abfallenden Volumenstrom. Dies wird dem Benutzer dadurch signalisiert, dass der Vibrationserreger 215 mit einer geänderten Intensität aktiviert wird, sobald der Volumenstrom das Minimum V'ₘᵢₙ unterschreitet.

Zurückkommend auf Figur 2 ist erkennbar, dass der gesamte Vernebler 200 hochgradig modular aufgebaut ist, und somit um Aufbau leicht variiert werden kann. So kann anstelle der geschlossenen Dosiereinrichtung 205 auch eine manuelle Dosierung eines Therapeutikums in den Aerosolgenetor 220 vorgesehen sein, dazu würde die Dosiereinrichtung 205 durch einen nicht dargestellten abnehmbaren Deckel ersetzt, welcher den Verneblerkopf 206 verschließt.

Der Verneblerkopf 206 ist zusätzlich so ausgeführt, dass der Aersolgenerator 220 zu Reinigungszwecken entnommen und/oder bei Verschleiß ausgetauscht werden kann. Gleichzeitig ist der Aerosolgenerator 220 als rotationssymmetrisches Bauteil ausgeführt, welches auch leicht in andere Systeme integriert werden kann, z.B. in stationäre Inhalations- oder Beatmungsgeräte.

Die vorstehend beschriebenen Ausführungsbeispiele dienen nur zur Erläuterung verschiedener Aspekte der Erfindung. Dabei können die einzelnen geschilderten Aspekte auch jeweils ohne Umsetzung anderer Aspekte einige der Aufgaben der Erfindung lösen, und die genannten Vorteile erreichen.

## Patentansprüche

1. Verneblersystem für einen Mesh-Vernebler (200) zur Erzeugung und Einbringung eines Aerosols in die Atemwege eines Patienten mit einer Basiseinheit (201) und wenigstens einem Mesh-Verneblerkopf (106, 206) und wenigstens einer Dosiereinheit (205), zur Bereitstellung einer vorgegebenen Menge eines Therapeutikums,
wobei das Therapeutikum durch die Dosiereinheit aus einem, mittels Druck zu öffnenden Portionsbehälter, dem Verneblerkopf zugeführt wird,
wobei die Dosiereinheit eine Aufnahmekammer mit einer Dosieröffnung zur Abgabe des Therapeutikums, einer Öffnung zum Einführen eines Portionsbehälters und einen Verschlusskörper für die Öffnung aufweist,
wobei der Verschlusskörper eingerichtet ist, von einer offenen Position, in welcher der Verschlusskörper die Öffnung freigibt, in einer geschlossene Position bewegt zu werden, in welcher der Verschlusskörper die Öffnung verschließt;
welche mit der Basiseinheit kombiniert werden können, um einen funktionsbereiten Vernebler zu bilden,
wobei der wenigstens eine Verneblerkopf (106, 206) mit einem Identifikationsmerkmal ausgerüstet ist,
wobei die Basiseinheit eine Steuerungseinheit umfasst, welche eingerichtet ist, das Identifikationsmerkmal des Verneblerkopfes (206) registrieren,
und wobei ferner die Steuerungseinrichtung eingerichtet ist, anhand des Identifikationsmerkmals festzustellen, ob der Betrieb des Verneblerkopfes (206) mit der Basiseinheit zulässig ist, und wobei das Identifikationsmerkmal einen Speicherchip umfasst, wobei die Basiseinheit und der Verneblerkopf (206) so ausgeführt sind, dass bei der Kombination der Basiseinheit mit dem Verneblerkopf (206) eine galvanische Verbindung zwischen der Steuerung und dem Speicherchip hergestellt wird.

2. Verneblersystem nach Anspruch 1, **dadurch gekennzeichnet, dass** die Steuerungseinheit eingerichtet ist, den Verneblerkopf (206) nur dann zu aktivieren, wenn diese mit der Basiseinheit verwendet werden darf.

3. Verneblersystem nach einem der vorangehenden Ansprüche, **dadurch gekennzeichnet, dass** das Identifikationsmerkmal Informationen über eine Haltbarkeit und/oder eine maximale Verwendungsdauer des Verneblerkopfes (206) beinhaltet

4. Verneblersystem nach Anspruch 3, **dadurch gekennzeichnet, dass** die Steuereinrichtung eingerichtet ist, Nutzungsdaten auf den RFID-Chip (720) oder den Speicherchip zurückzuschreiben.

## Claims

1. Nebulizer system for a mesh nebulizer (200) for generating and introducing an aerosol into the airways of a patient with
a base unit (201) and at least one mesh-nebulizer head (106, 206) and at least one dosing unit (205), for providing a predetermined amount of a therapeutic agent,
wherein the therapeutic agent is supplied to the nebulizer head by the dosing unit from a portion container which can be opened by means of pressure,
wherein the dosing unit has an accommodation chamber with a dosing opening for dispensing the therapeutic agent, an opening for introducing a portion container and a locking body for the opening,
wherein the locking body is adapted to be moved from an open position, in which the locking body exposes the opening, to a closed position, in which the locking body closes the opening; which can be combined with the base unit to form a functional nebulizer,
wherein the at least one nebulizer head (106, 206) is equipped with an identification attribute, wherein the base unit comprises a control unit which is adapted to register the identification attribute of the nebulizer head (206) and wherein the control unit is configured to determine, on the basis of the identification attribute, whether the operation of the nebulizer head (206) with the base unit is permissible, and wherein the identification attribute comprises a memory chip, wherein the base unit and the nebulizer head (206) are designed such that a galvanic connection is established between the control unit and the memory chip when the base unit is combined with the nebulizer head (206).

2. Nebulizer system according to claim 1, **characterized in that** the control unit is arranged to activate the nebulizer head (206) only when use with the base unit is allowed.

3. Nebulizer system according to one of the preceding claims, **characterized in that** the identification attribute contains information about a shelf life and/or a maximum period of use of the nebulizer head (206).

4. Nebulizer system according to claim 3, **characterized in that** the control device is set up to write usage data back to the RFID chip (720) or the memory chip.

## Revendications

1. Système de nébuliseur pour un nébuliseur à mailles (200) destiné à produire et à introduire un aérosol dans les voies respiratoires d'un patient, comprenant une unité de base (201) et au moins une tête de nébuliseur à mailles (106, 206) et au moins une unité de dosage (205) pour fournir une quantité prédéterminée d'un agent thérapeutique,
dans lequel l'agent thérapeutique est amené à la tête de nébuliseur par l'unité de dosage à partir d'un récipient de portions pouvant être ouvert par pression, dans lequel l'unité de dosage comprend une chambre de réception avec une ouverture de dosage pour distribuer le produit thérapeutique, une ouverture pour introduire un récipient à portions et un corps de fermeture pour l'ouverture,
dans lequel le corps de fermeture est agencé pour être déplacé d'une position ouverte, dans laquelle le corps de fermeture libère l'ouverture, à une position fermée, dans laquelle le corps de fermeture ferme l'ouverture ; lesquels peuvent être combinés avec l'unité de base pour former un nébuliseur prêt à fonctionner, dans lequel l'au moins une tête de nébuliseur (106, 206) est équipée d'une caractéristique d'identification,
dans lequel l'unité de base comprend une unité de commande qui est agencée pour enregistrer la caractéristique d'identification de la tête de nébuliseur (206),
et dans lequel en outre le dispositif de commande est agencé pour déterminer, à l'aide de la caractéristique d'identification, si le fonctionnement de la tête de nébuliseur (206) est autorisé avec l'unité de base, et dans lequel la caractéristique d'identification comprend une puce de mémoire, dans lequel l'unité de base et la tête de nébuliseur (206) sont réalisées de telle sorte que, lors de la combinaison de l'unité de base avec la tête de nébuliseur (206), une liaison galvanique est établie entre la commande et la puce de mémoire.

2. Système de nébuliseur selon la revendication 1, **caractérisé en ce que** l'unité de commande est agencée pour activer la tête de nébuliseur (206) uniquement lorsque celle-ci peut être utilisée avec l'unité de base.

3. Système de nébuliseur selon l'une des revendications précédentes, **caractérisé en ce que** la caractéristique d'identification comprend des informations sur une durée de conservation et/ou une durée d'utilisation maximale de la tête de nébuliseur (206).

4. Système de nébuliseur selon la revendication 3, **caractérisé en ce que** le dispositif de commande est agencé pour réécrire des données d'utilisation sur la puce RFID (720) ou sur la puce de mémoire.
